# EUROPEAN PATENT APPLICATION

(11) **EP 1 674 855 A2**
(43) Date of publication of application: **28.06.2006**
(21) Application number: 05024148.8
(22) Date of filing: 04.11.2005
(51) Int. Cl.: G01N 21/55, G01N 30/72

(54) **Addressable recovery of bound analytes from an evanescent wave sensor**

(30) Priority: 15.12.2004 US 13635
(71) Applicant: AGILENT TECHNOLOGIES, INC. (A Delaware Corporation), Palo Alto, CA 94306-2024 (US)
(72) Inventor: Robotti, Karla M., Mountain View, CA 94043 (US); Roitman, Daniel, Menlo Park, CA 94025 (US)
(74) Representative: Schoppe, Fritz

(57) **Abstract**

The invention provides an evanescent wave sensor containing a cleavably bound capture agent. Upon exposure of the sensor to a suitable stimulus the capture agent and any analyte bound thereto are released from the sensor. The released analyte may be transferred into a mass spectrometer apparatus and investigated therein.

## Description

### BACKGROUND OF THE INVENTION

Sensitive and accurate methods for detecting molecular interactions are very desirable for a wide variety of applications, including drug discovery, environmental testing, diagnostics, gene expression analysis, genomics analysis, proteomics and for characterizing the binding of two molecules that are known to bind together.

In representative methods for detecting molecular interactions, target molecules (i.e., molecules of interest that may be present in a sample) are labeled with a radioactive or optically detectable (e.g., a light-emitting or colorigenic) moiety and contacted with molecular probes for the target molecules under specific binding conditions. In these methods, binding of target molecules to the probes for those target molecules can be determined by assessing the amount of label associated with the probes. While these methods have found general use in many laboratories, their use is limited because they may only be employed in methods in which it is possible to label the target molecules. Furthermore, because labeling often requires relatively complex procedures and can result in inefficient or biased labeling, such detection methods may be complicated, insensitive and may produce unreliable results.

One type of label-free method for detecting molecular interactions exploits a surface-sensitive physical phenomenon called an evanescent wave. Such methods detect total internal reflection of light at a surface-solution interface that produces an electromagnetic field (an evanescent wave), extending a short distance (typically in the order of hundreds of nanometers) into the solution. The evanescent wave that occurs outside of a totally internally reflecting prism is sensitive to refractive index changes in the solution in contact with surface of the prism, and when an analyte binds to the outside of the reflective surface of the prism, the effective refractive index change occurs on the solution side because the evanescent wave probes the solution very near this interface. Thus, binding of an analyte can be detected by detecting changes in the degree of total internal reflection and the amplitude of the reflected light.

These evanescent wave methods, including surface plasmon resonance methods, usually employ a prism having a planar coating of metal (e.g., gold) on the reflective side of a dielectric material, e.g., a glass, quartz or plastic prism. Capture agents, e.g., peptides, antibodies or other chemical moieties, are usually linked to the metal coating, an aqueous solution containing analyte molecules is passed over the capture agents, and binding of the analyte molecules to the capture agents is detected.

Although such methods have become well used in the research community, their use is limited because there is no way of investigating the identity of an analyte bound to a capture agent. In other words, while an evanescent wave sensor may be able to detect binding of a capture agent to an analyte, such a sensor cannot be used to investigate (e.g., confirm or elucidate) the identity of the bound analyte.

Accordingly, there is a great need for improved evanescent wave sensors. In particular, there is a great need for an evanescent wave sensor that facilitates investigations into the structure of any analytes detected thereon.

The invention described herein meets this need, and others.

### SUMMARY OF THE INVENTION

The invention provides an evanescent wave sensor containing a cleavably bound capture agent. Upon exposure of the sensor to a suitable stimulus, e.g., light, the capture agent and any analyte bound thereto are released from the sensor. The released analyte may be transferred into a mass spectrometer apparatus and investigated therein. Also provided by the invention are methods in which a subject evanescent wave sensor is contacted with a sample, and binding of analytes in the sample to the sensor is assessed by both evanescent wave detection and mass spectrometry. The invention also provides kits and systems for performing the subject methods. The invention finds use in a variety of applications in which it is desirable to detect analytes, e.g., drug discovery, environmental and diagnostic applications.

### BRIEF DESCRIPTION OF THE DRAWINGS

The invention is best understood from the following detailed description when read in conjunction with the accompanying drawings. It is emphasized that, according to common practice, the various features of the drawings are not to-scale. On the contrary, the dimensions of the various features are arbitrarily expanded or reduced for clarity. Included in the drawings are the following figures:

Figs. 1A-1B schematically illustrates exemplary evanescent wave sensor devices of the present invention.

Fig. 2 shows an ortho-nitrobenzyl group present in exemplary photocleavable linkers employed in the present invention.

Fig. 3A-3M show exemplary cleavable linkers containing ortho-nitrobenzyl groups.

Fig. 4 schematically illustrates an exemplary analyte detection system of the present invention.

Fig. 5 schematically illustrates an exemplary method of analyte detection employing an evanescent wave sensor of the present invention.

Figs. 6A-6C schematically illustrates exemplary results obtained using a method of analyte detection of the present invention.

### DEFINITIONS

Before the present invention is further described, it is to be understood that this invention is not limited to particular embodiments described, as such may of course vary. It is also to be understood that the terminology used herein is for the purpose of describing particular embodiments only, and is not intended to be limiting. Unless defined otherwise, all technical and scientific terms used herein have the same meaning as commonly understood by one of ordinary skill in the art to which this invention belongs.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Throughout this application, various publications, patents and published patent applications are cited. The disclosures of these publications, patents and published patent applications referenced in this application are hereby incorporated by reference in their entirety into the present disclosure. Citation herein by Applicant of a publication, patent, or published patent application is not an admission by Applicant of said publication, patent, or published patent application as prior art.

It must be noted that as used herein and in the appended claims, the singular forms "a", "and", and "the" include plural referents unless the context clearly dictates otherwise. Thus, for example, reference to "a layer" includes a plurality of such layers, and reference to "the capture agent" includes reference to one or more capture agent and equivalents thereof known to those skilled in the art, and so forth. It is further noted that the claims may be drafted to exclude any optional element. As such, this statement is intended to serve as antecedent basis for use of such exclusive terminology as "solely", "only" and the like in connection with the recitation of claim elements, or the use of a "negative" limitation.

The term "sample" as used herein relates to a material or mixture of materials, typically, although not necessarily, in fluid form, e.g., aqueous or in solvent, containing one or more components of interest. Samples may be derived from a variety of sources such as from food stuffs, environmental materials, a biological sample such as tissue or fluid isolated from an individual, including but not limited to, for example, plasma, serum, spinal fluid, semen, lymph fluid, the external sections of the skin, respiratory, intestinal, and genitourinary tracts, tears, saliva, milk, blood cells, tumors, organs, and also samples of *in vitro* cell culture constituents (including but not limited to conditioned medium resulting from the growth of cells in cell culture medium, putatively virally infected cells, recombinant cells, and cell components).

Components in a sample are termed "analytes" herein. In many embodiments, the sample is a complex sample containing at least about 10², 5x10², 10³, 5x10³, 10⁴, 5x10⁴, 10⁵, 5x10⁵, 10⁶, 5x10⁶, 10⁷, 5x10⁷, 10⁸, 10⁹, 10¹⁰, 10¹¹, 10¹² or more species of analyte. In certain embodiments, a sample may contain a purified analyte.

The term "analyte" is used herein to refer to a known or unknown component of a sample, which will specifically bind to a capture agent on a substrate surface if the analyte and the capture agent are members of a specific binding pair. In general, analytes are chemical molecules of interest, e.g., biopolymers, i.e., an oligomer or polymer such as an oligonucleotide, a peptide, a polypeptide, an antibody, or the like. In this case, an "analyte" is referenced as a moiety in a mobile phase (typically fluid), to be detected by a "capture agent" which is bound to a substrate. However, either of the "analyte" or "capture agent" may be the one which is to be evaluated by the other (thus, either one could be an unknown mixture of analytes, e.g., polypeptides, to be evaluated by binding with the other).

A "biopolymer" is a polymer of one or more types of repeating units, regardless of the source (e.g., biological (e.g., naturally-occurring, obtained from a cell-based recombinant expression system, and the like) or synthetic). Biopolymers may be found in biological systems and particularly include polypeptides and polynucleotides, including compounds containing amino acids, nucleotides, or a mixture thereof.

The terms "polypeptide" and "protein" are used interchangeably throughout the application and mean at least two covalently attached amino acids, which includes proteins, polypeptides, oligopeptides and peptides. A polypeptide may be made up of naturally occurring amino acids and peptide bonds, synthetic peptidomimetic structures, or a mixture thereof. Thus "amino acid", or "peptide residue", as used herein encompasses both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the D- or the L- configuration.

In general, biopolymers, e.g., polypeptides or polynucleotides, may be of any length, e.g., greater than 2 monomers, greater than 4 monomers, greater than about 10 monomers, greater than about 20 monomers, greater than about 50 monomers, greater than about 100 monomers, greater than about 300 monomers, usually up to about 500, 1000 or 10,000 or more monomers in length. "Peptides" and "oligonucleotides" are generally greater than 2 monomers, greater than 4 monomers, greater than about 10 monomers, greater than about 20 monomers, usually up to about 10, 20, 3 0, 40, 50 or 100 monomers in length. In certain embodiments, peptides and oligonucleotides are between 5 and 30 amino acids in length.

The terms "polypeptide" and "protein" are used interchangeably herein. The term "polypeptide" includes polypeptides in which the conventional backbone has been replaced with non-naturally occurring or synthetic backbones, and peptides in which one or more of the conventional amino acids have been replaced with one or more non-naturally occurring or synthetic amino acids. The term "fusion protein" or grammatical equivalents thereof references a protein composed of a plurality of polypeptide components, that while typically not attached in their native state, typically are joined by their respective amino and carboxyl termini through a peptide linkage to form a single continuous polypeptide. Fusion proteins may be a combination of two, three or even four or more different proteins. The term polypeptide includes fusion proteins, including, but not limited to, fusion proteins with a heterologous amino acid sequence, fusions with heterologous and homologous leader sequences, with or without N-terminal methionine residues; immunologically tagged proteins; fusion proteins with detectable fusion partners, e.g., fusion proteins including as a fusion partner a fluorescent protein, β-galactosidase, luciferase, and the like.

The term "capture agent" refers to an agent that binds an analyte through an interaction that is sufficient to permit the agent to bind and concentrate the analyte from a homogeneous mixture of different analytes. The binding interaction is typically mediated by an affinity region of the capture agent. Typical capture agents include any moiety that can specifically bind to an analyte. In certain embodiments, a polypeptide, e.g., a monoclonal antibody or a peptide, may be employed. Capture agents usually "specifically bind" one or more analytes.

Accordingly, the term "capture agent" refers to a molecule or a multi-molecular complex which can specifically bind an analyte, e.g., specifically bind an analyte for the capture agent, with a dissociation constant (K_{D}) of less than about 10⁻⁴ M (e.g., less than about 10⁻⁶ M) without binding to other targets.

The term "specific binding" refers to the ability of a capture agent to preferentially bind to a particular analyte that is present in a homogeneous mixture of different analytes. Typically, a specific binding interaction will discriminate between desirable and undesirable analytes in a sample, typically more than about 10 to 100-fold or more (e.g., more than about 1004- or 10,000-fold). Typically, the affinity between a capture agent and analyte when they are specifically bound in a capture agent/analyte complex is characterized by a K_{D} (dissociation constant) of at least 10⁻⁴ M, at least 10⁻⁵ M, at least 10⁻⁶ M, at least 10⁻⁷ M, at least 10⁻⁸ M, at least 10⁻⁹ M, usually up to about 10⁻¹⁰ M.

The term "capture agent/analyte complex" is a complex that results from the specific binding of a capture agent with an analyte, i.e., a "binding partner pair". A capture agent and an analyte for the capture agent will usually specifically bind to each other under "conditions suitable for specific binding", where such conditions are those conditions (in terms of salt concentration, pH, detergent, protein concentration, temperature, etc.) which allow for binding to occur between capture agents and analytes to bind in solution. Such conditions, particularly with respect to proteins and nucleic acids are well known in the art (see, e.g., Harlow and Lane (Antibodies: A Laboratory Manual Cold Spring Harbor Laboratory, Cold Spring Harbor, N.Y. (1989) and Ausubel, et al, Short Protocols in Molecular Biology, 5th ed., Wiley & Sons, 2002). Conditions suitable for specific binding typically permit capture agents and target pairs that have a dissociation constant (KD) of less than about 10⁻⁶ M to bind to each other, but not with other capture agents or targets. "Hybridizing" and "binding", with respect to polynucleotides, are used interchangeably.

As used herein, "binding partners" and equivalents refer to pairs of molecules that can be found in a capture agent/analyte complex, i.e., exhibit specific binding with each other.

The phrase "surface-bound capture agent" refers to a capture agent that is immobilized on a surface of a solid substrate. In certain embodiments, the capture agents employed herein are present on a surface of the same support, e.g., a subject sensor.

The term "pre-determined" refers to an element whose identity is known prior to its use. For example, a "pre-determined analyte" is an analyte whose identity is known prior to any binding to a capture agent. An element may be known by name, sequence, molecular weight, its function, or any other attribute or identifier. In some embodiments, the term "analyte of interest", i.e., an known analyte that is of interest, is used synonymously with the term "pre-determined analyte".

The terms "antibody" and "immunoglobulin" are used interchangeably herein to refer to a capture agent that has at least an epitope binding domain of an antibody. These terms are well understood by those in the field, and refer to a protein containing one or more polypeptides that specifically binds an antigen. One form of antibody constitutes the basic structural unit of an antibody. This form is a tetramer and consists of two identical pairs of antibody chains, each pair having one light and one heavy chain. In each pair, the light and heavy chain variable regions are together responsible for binding to an antigen, and the constant regions are responsible for the antibody effector functions. Types of antibodies, including antibody isotypes, monoclonal antibodies and antigen-binding fragments thereof (e.g., Fab, Fv, scFv, and Fd fragments, chimeric antibodies, humanized antibodies, single-chain antibodies, etc) are well known in the art and need not be described in any further detail.

An "array," includes any one, two-dimensional or substantially two-dimensional (as well as a three-dimensional) arrangement of addressable regions bearing a particular chemical moiety or moieties (e.g., biopolymers such as polynucleotide or oligonucleotide sequences (nucleic acids), polypeptides (e.g., proteins), carbohydrates, lipids, etc.) associated with that region. In the broadest sense, the preferred arrays are arrays of polymeric binding agents, where the polymeric binding agents may be any of: polypeptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like. Where the arrays are arrays of nucleic acids, the nucleic acids may be attached to the arrays at any point along the nucleic acid chain, but are generally attached at one of their termini (e.g. the 3' or 5' terminus). Sometimes, the arrays are arrays of polypeptides, e.g., proteins or fragments thereof.

Any given substrate may carry one, two, four or more or more arrays disposed on a front surface of the substrate. Depending upon the use, any or all of the arrays may be the same or different from one another and each may contain multiple spots or features. A typical array may contain more than ten, more than one hundred, more than one thousand more ten thousand features, or even more than one hundred thousand features, in an area of less than 20 cm² or even less than 10 cm². For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents but may not be present when, for example, photolithographic array fabrication processes are used. It will be appreciated though, that the interfeature areas, when present, could be of various sizes and configurations.

Each array may cover an area of less than 100 cm², or even less than 50 cm², 10 cm² or 1 cm². In many embodiments, the substrate carrying the one or more arrays will be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate 10 may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse jets of either precursor units (such as amino acid or nucleotide monomers) in the case of in situ fabrication, or the previously obtained polymer. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. As already mentioned, these references are incorporated herein by reference. Other drop deposition methods can be used for fabrication, as previously described herein. Also, instead of drop deposition methods, photolithographic array fabrication methods may be used. Interfeature areas need not be present particularly when the arrays are made by photolithographic methods.

An array is "addressable" when it has multiple regions of different moieties (e.g., different polynucleotide sequences) such that a region (i.e., a "feature" or "spot" of the array) at a particular predetermined location (i.e., an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probe" may be the one which is to be evaluated by the other (thus, either one could be an unknown mixture of polynucleotides to be evaluated by binding with the other). A "scan region" refers to a contiguous (preferably, rectangular) area in which the array spots or features of interest, as defined above, are found. The scan region is that portion of the total area illuminated from which the resulting fluorescence is detected and recorded. For the purposes of this invention, the scan region includes the entire area of the slide scanned in each pass of the lens, between the first feature of interest, and the last feature of interest, even if there exist intervening areas which lack features of interest. An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

The term "mixture", as used herein, refers to a combination of elements, e.g., capture agents or analytes, that are interspersed and not in any particular order. A mixture is homogeneous and not spatially separated into its different constituents. Examples of mixtures of elements include a number of different elements that are dissolved in the same aqueous solution, or a number of different elements attached to a solid support at random or in no particular order in which the different elements are not specially distinct. In other words, a mixture is not addressable. To be specific, an array of capture agents, as is commonly known in the art, is not a mixture of capture agents because the species of capture agents on an array are spatially distinct and addressable.

"Isolated" or "purified" generally refers to isolation of a substance (compound, polynucleotide, protein, polypeptide, polypeptide composition) such that the substance comprises a significant percent (e.g., greater than 2%, greater than 5%, greater than 10%, greater than 20%, greater than 50%, or more, usually up to about 90%-100%) of the sample in which it resides. In certain embodiments, a substantially purified component comprises at least 50%, 80%-85%, or 90-95% of the sample. Techniques for purifying polynucleotides and polypeptides of interest are well-known in the art and include, for example, ion-exchange chromatography, affinity chromatography and sedimentation according to density. Generally, a substance is purified when it exists in a sample in an amount, relative to other components of the sample, that is not found naturally.

The term "assessing" includes any form of measurement, and includes determining if an element is present or not. The terms "determining", "measuring", "evaluating", "assessing" and "assaying" are used interchangeably and may include quantitative and/or qualitative determinations. Assessing may be relative or absolute. "Assessing the presence of" includes determining the amount of something present, and/or determining whether it is present or absent.

The term "using" has its conventional meaning, and, as such, means employing, e.g., putting into service, a method or composition to attain an end. For example, if a program is used to create a file, a program is executed to make a file, the file usually being the output of the program. In another example, if a computer file is used, it is usually accessed, read, and the information stored in the file employed to attain an end. Similarly if a unique identifier, e.g., a barcode is used, the unique identifier is usually read to identify, for example, an object or file associated with the unique identifier.

By "remote location," it is meant a location other than the location at which the sensor is present and binding occurs. For example, a remote location could be another location (e.g., office, lab, *etc.)* in the same city, another location in a different city, another location in a different state, another location in a different country, *etc.* As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An sensor "package" may contain only the sensor, although the package may include other features (such as a housing with a chamber). It will also be appreciated that throughout the present application, that words such as "top," "upper," and "lower" are used in a relative sense only.

A "computer-based system" refers to the hardware means, software means, and data storage means used to analyze the information in accordance with the invention. The minimum hardware of the computer-based systems typically comprises a central processing unit (CPU), input means, output means, and data storage means. A skilled artisan can readily appreciate that any one of the currently available computer-based system are suitable for use in embodiments in accordance with the invention. The data storage means may comprise any manufacture comprising a recording of the information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, *etc.*

A "processor" references any hardware and/or software combination that will perform the functions required of it. For example, any processor herein may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

If one compositions is "bound" to another composition, the compositions do not have to be in direct contact with each other. In other words, bonding may be direct or indirect, and, as such, if two compositions (e.g., a substrate and a capture agent) are bound to each other, there may be at least one other composition (e.g., another layer) between to those compositions. Binding between any two compositions described herein may be covalent or non-covalent. The terms "bound" and "linked" are used interchangeably herein.

A "prism" is an transparent body that is bounded in part by two nonparallel plane faces and is used to refract or disperse a beam of light. The term prism encompasses round, cylindrical-plane lenses (e.g., semicircular cylinders) and a plurality of prisms in contact with each other.

Other definitions of terms appear throughout the specification.

### DETAILED DESCRIPTION OF THE INVENTION

The invention provides an evanescent wave sensor containing a cleavably bound capture agent. Upon exposure of the sensor to a suitable stimulus, e.g., light, the capture agent and any analyte bound thereto are released from the sensor. The released analyte may be transferred into a mass spectrometer apparatus and investigated therein. Also provided by the invention are methods in which a subject evanescent wave sensor is contacted with a sample, and binding of analytes in the sample to the sensor is assessed by both evanescent wave detection and mass spectrometry. The invention also provides kits and systems for performing the subject methods. The invention finds use in a variety of applications in which it is desirable to detect analytes, e.g., drug discovery, environmental and diagnostic applications. For example, the invention may be employed to investigate post-translational modifications, point mutations and for epitope mapping.

In describing the invention in greater detail than provided in the Summary and as informed by the Background and Definitions provided above, the subject evanescent wave sensors are described first, followed by a description of an analyte detection system employing a subject evanescent wave sensor. Following this, a discussion of methods of using a subject evanescent wave sensor to detect an analyte will be presented. Finally, kits for performing the subject methods are described.

### EVANESCENT WAVE SENSORS

As mentioned above, the invention provides an evanescent wave sensor containing a cleavably-bound capture agent where a "cleavably bound capture agent" is a substrate-bound capture agent that is releasable from the substrate to which it is bound by exposing the bound capture agent to a cleavage-inducing stimulus. The stimulus breaks a covalent bond that links the capture agent to the substrate and releases the capture agent from the substrate. The stimulus does not break covalent bonds within the capture agent, or any analyte bound thereto. Depending on the particular chemistry used and as described in greater detail below, the stimulus may be directed energy (e.g., light provided by a laser, for example) or a chemical, for example.

A subject cleavable linker-containing evanescent wave sensor may be employed in a method of detecting an analyte in which an analyte is: bound to a subject evanescent wave sensor and detected thereby, cleaved from the sensor after it is detected, and transported to a second detection device, e.g., a mass spectrometer, where it is analyzed. In many embodiments, this method includes: a) contacting a sample with a subject sensor, b) assessing the presence of an analyte bound to the sensor by detecting an evanescent wave; c) cleaving the capture agent from the sensor to release the capture agent and bound analyte; and d) transporting the analyte to a mass spectrometry apparatus for analysis.

Accordingly, in one embodiment, a subject evanescent wave sensor may be operably linked with a mass spectrometer apparatus to provide an integrated analyte detection system. That system may be employed to a) detect binding of an analyte to a capture agent by detecting an evanescent wave and b) further investigate the identity of the bound analyte by mass spectrometry. These methods will be described in greater detail below.

In describing the subject cleavable linker-containing evanescent wave sensors, an overview of the subject sensors will be presented first, followed by a description of cleavable linkers suitable for use in the subject sensors.

A subject evanescent wave sensor may be adapted for use in a particular type of detection method, e.g., a surface plasmon resonance method, and, as such, may be dimensioned and made of materials suitable for that method. Since many evanescent wave detection methods are generally well known in the art (e.g., surface plasmon resonance, grating coupler surface plasmon resonance, resonance mirror sensing and waveguide sensor interferometry using Mach-Zender or polarimetric methods, direct and indirect evanescent wave detection methods etc.; see also Myszka J. Mol. Rec. 1999 12:390-408 and *Biomolecular Sensors,* edited by Gizeli and Lowe. Taylor & Francis, May 2002), one of skill in the art would know how to adapt a subject sensor for use in particular method without undue effort. Exemplary surface plasmon resonance methods will be described in greater detail below.

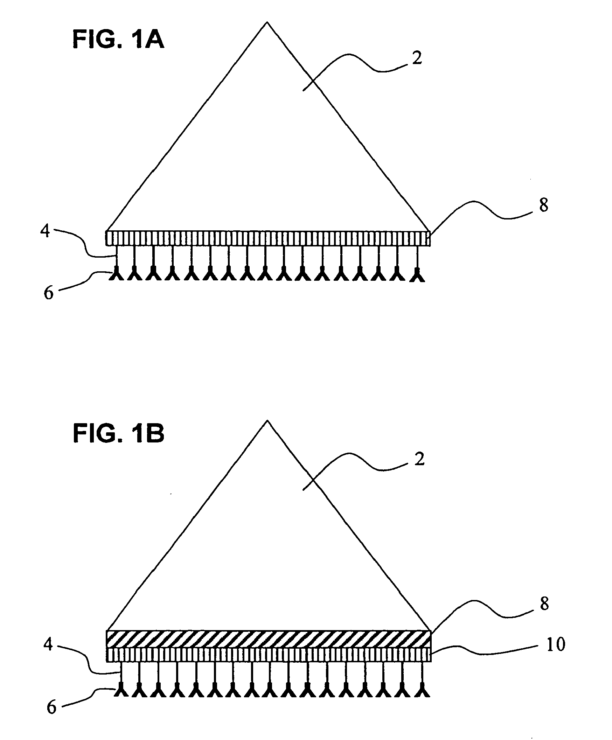

With reference to Fig. 1A, a subject sensor may contain at least three components, represented by prism 2, cleavable linker 4 and capture agent 6. Element 8, a metal layer, may be present between cleavable linker 4 and prism 2 in certain embodiments. Prism 2 and metal layer 8 are well known components of many known evanescent wave sensors and need not be described herein in any great detail. Prism 2 has in certain embodiments at least one planar surface and is transparent to the particular light beam used for detection. Prism **2** may be made of a dielectric, transparent material (i.e., a material transparent to light at wavelengths used for analyte detection using the subject sensor) having a refractive index (n) of about 0.5 to about 1.5, e.g., of about 0.6, about 0.7, about 0.8, about 0.9, about 1.0, about 1.2, or about 1.4. Glass, quartz, silica, and plastic and moldable materials, such as cyclic olefin (e.g., TOPAS™ or ZEONOR™), polyolefin, polydimethylsiloxane (PDMS), polymethylmethyl acrylate (PMMA; e.g., LUCITE™ or PLEXIGLASS™) are suitable materials for making a prism. In particular embodiments, the refractive index of the substrate is matched to that of the sample to be analyzed using the subject methods. Further, a layer of dielectric material (e.g., TiO₂ or SiO₂ the like), of about 300-600nm thickness (e.g., about 400 nm) may be present between metal layer 8, if present, and prism 2. Such dielectric materials and their use in evanescent wave sensors are well known in the art, and, used herein, may sharpen resonance peaks, serve as an adhesion layer, and protect any metal layer during fabrication.

One planar surface of the substrate 2 may be coated in a film of metal 8, usually a free electron metal such as, e.g., copper, silver, aluminum or gold, although other metals may be used. As is well known in the art, different metals produce different resonance effects, and, as such, the choice of metal depends on the resonance effect desired. This metal coating may be produced using known methods, e.g., sputter or coating, and its thickness may be in the range of about 200Å to about 600Å. In particular embodiments, the substrate is coated in gold, which is well known in surface plasmon resonance detectors. A metal grating, as is commonly used in certain surface plasmon resonance methods, may also be present in a subject sensor.

The cleavable linker 4 may optionally be linked to the prism 2 via a hydrogel (e.g., a hydrogel film on the surface of prism 2), as described in U.S. Patent 5,242,828 and Fong et al. (Analytica Chimica Acta 2002 456:201-208), which references are incorporated herein in their entirety.

Fig. 1B illustrates a further embodiment of the invention. Fig. 1B shows a sensor having at least four components, represented by prism 2, cleavable linker 4, capture agent 6 substrate 10, and metal layer 8. In this sensor, prism 2 is affixed to substrate 10 such that light can pass through the prism 2 and substrate 10 and reflect off optional metal layer 8. The refractive indices of prism 2 and substrate 10 may be matched. The arrangement of elements shown in Fig. 1B is most often used in evanescent wave detectors. As mentioned above, a layer of dielectric material may be present between the metal layer, if present, and the cleavable linkers 4. Also as discussed above, cleavable linker 4 may optionally be linked to the substrate 10 via a hydrogel matrix.

In many embodiments, a substrate employed in a subject sensor may betransparent to the light used to generate the evanescent wave. For example, in certain embodiments, a substrate may be transparent to monochromatic light having a wavelength in the range of about 0.6 µm to about 2.0 µm, e.g., about 0.8 µm to about 1.7 µm, about 0.8 µm to about 1.4 µm, etc., depending on the light beam used in the subject methods. For example, a subject substrate may be transparent to monochromatic light having a wavelength of about 0.6 µm, about 0.7 µm, about 0.8 µm, about 0.9 µm, about 1.0 µm, about 1.1 µm, about 1.2 µm, about 1.3 µm, about 1.4 µm, about 1.5 µm, about 1.6 µm, about 1.7 µm, about 1.8 µm or greater, or a range of these wavelengths.

A subject sensor may contain an array of capture agents.

### Cleavable linkers

As mentioned above, a subject sensor contains capture agents that are cleavably bound (i.e., bound indirectly or directly) to the surface of a substrate. In many embodiments, the capture agent is bound to the substrate via a cleavable linker.

Cleavable linkers that may be employed in the subject methods include electrophilically cleavable linkers, nucleophilically cleavable linkers, photocleavable linkers, metal cleavable linkers, electrolytically-cleavable, and linkers that are cleavable under reductive and oxidative conditions. Such linkers are described in great detail by Guillier et al (Chem. Rev. 2000 1000:2091-2157), which disclosure is incorporated by reference in its entirety.

In particular embodiments, a photocleavable linker (e.g., a uv-cleavable linker) may be employed in a subject evanescent wave sensor. Suitable photocleavable linkers for use in a subject sensor include ortho-nitrobenzyl-based linkers, phenacyl linkers, alkoxybenzoin linkers, chromium arene complex linkers, NpSSMpact linkers and pivaloylglycol linkers, as described in Guillier et al, *supra.*

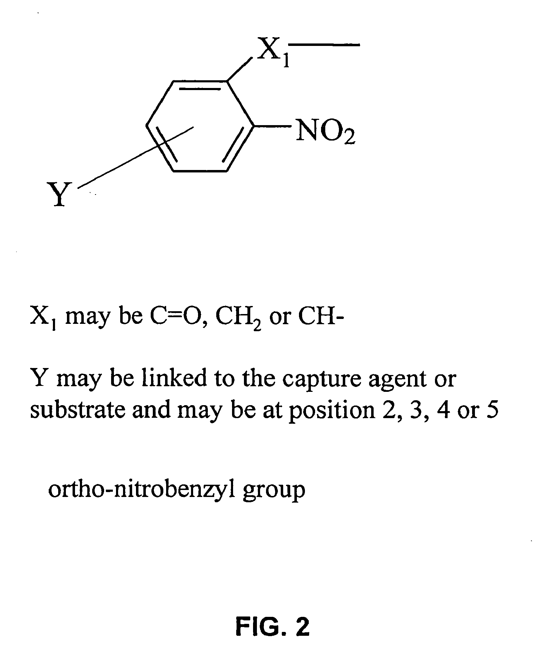

Of the photocleavable linkers, ortho-nitrobenzyl-based linkers are of particular interest since they can be made with a high yield and can be straightforwardly protected from cleavage-stimulating light (e.g., ultraviolet light, or "uv" light, i.e., light having a wavelength of 100 and 400 nm, e.g., UVA light (315-400 nm), UVB light (280-315 nm), UVC light (200-280 nm) or VUV light (100-200 nm)). Ortho-nitrobenzyl-based linkers contain an ortho-nitrobenzyl group (Fig. 2). Ortho-nitrobenzyl-based linkers, suitable methods for their synthesis and their use in photocleavably linking biomolecules to a substrate are discussed in great detail by Guillier et al, *supra* and Olejnik et al (Methods in Enzymology 1998 291:135-154), and further described in U.S.P.N. 6,027,890; Olejnik et al (Proc. Natl. Acad Sci, 92:7590-94 ); Ogata et al. (Anal. Chem. 2002 74:4702-4708); Bai et al (Nucl. Acids Res. 2004 32:535-541); Zhao et al (Anal. Chem. 2002 74:4259-4268); and Sanford et al (Chem Mater. 1998 10:1510-20), and, accordingly, are well known in the art and need not be described here in great detail.

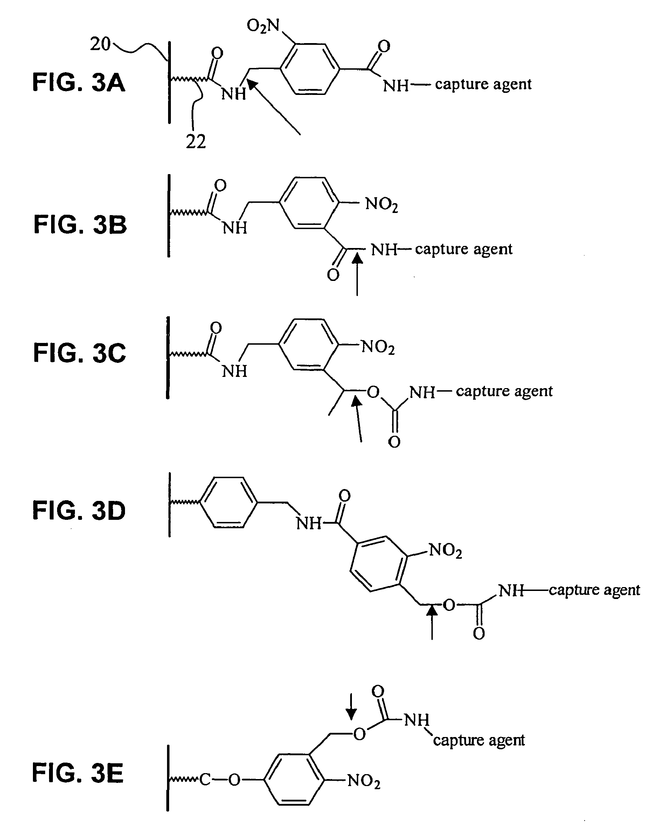

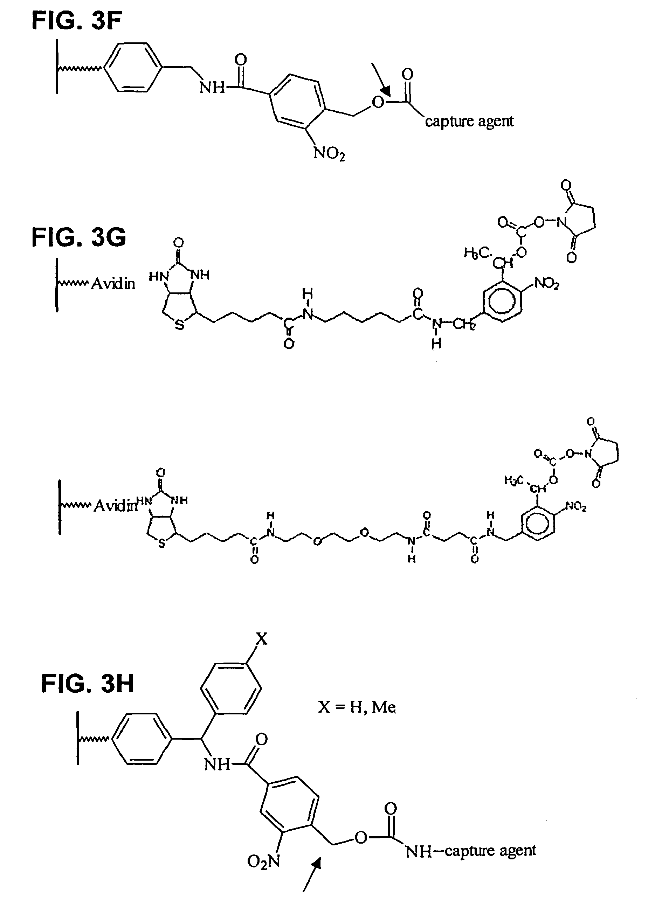

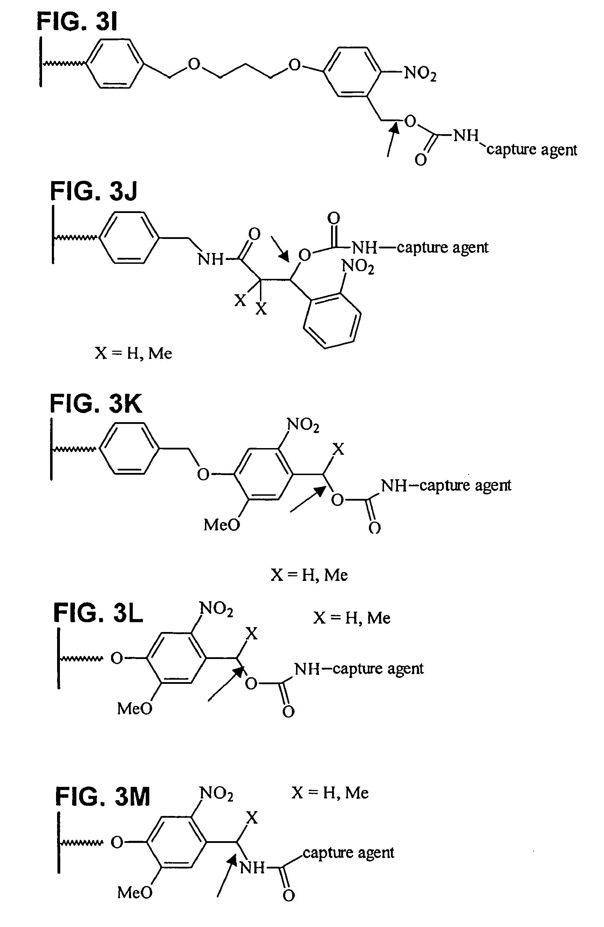

A wide variety of ortho-nitrobenzyl-based linkers are known in the art and are available for use in the subject methods. Fig. 3A-3M shows the chemical structures of a few of those linkers, and illustrates a capture agent covalently linked to the ortho-nitrobenzyl-group linker via an amino linkage (although other linkages are readily employed). The ortho-nitrobenzyl-group is tethered to substrate 20 via linker 22. The arrow on each of these figures indicates the covalent bond that is light-cleavable. As is known in the art and depending on the chemistry used, linker 22 and substrate 20 may be linked via a covalent or non-covalent bond (e.g., via binding of biotin to streptavidin or avidin).

As is known in the art, a capture agent can be tethered to a substrate using a suitable linking agent (e.g., a suitable ortho-nitrobenzyl-based linking agent) that generally possesses the following features, in order: a tag for linking to a substrate, a spacer moiety, a cleavable linker and a reactive group. The tag may be an affinity tag, e.g., a biotin group or the like, or a reactive moeity (e.g. a carboxy group, an amino group, a halo group, a tosylate group, a mesylate group, a reactive hydroxyl groups or metal oxide ) that can react with suitable sites (e.g., alcohols, amino nucleophliles, thiol nucleophiles or silane groups on the surface of a substrate to produce a covalent bond between the substrate and the linker. The spacer may contain an unreactive alkyl chain, e.g., containing 3-12 carbon atoms (e.g., 5-aminocaproic acid) and the cleavable linker may be chosen as containing appropriate chemistry (see above). The reactive group generally reacts with the capture agent and forms a covalent bond therewith. The reactive group is selectively reactive with particular chemical groups in the capture agent. Suitable reactive groups include halogens (that are sulhydryl reactive), N-hydroxysuccinimide (NHS)-carbonate (that are amine-reactive) and N,N-diisopropyl-2-cyanoethyl phosphoramidite (that are hydroxyl-reactive), and several other reactive groups are known in the art and may be readily employed in the instant methods.

In particular embodiments, it is desirable to cleave a cleavably-bound capture agent molecule at a bond that releases a "native" form of the capture agent, i.e., a capture agent that has an identical molecular structure to that of the same capture agent prior to becoming linked to the cleavable linker. In these embodiments, a photocleavable ortho-nitrobenzyl NHS linking agent may be employed. In these embodiments, at least one amino group (NH₂) of a capture agent is reacted with the NHS group of the linking agent to produce a molecule in which the linker and the capture agent are covalently linked via a uv-cleavable bond. Upon subjecting this molecule to uv light, the uv-cleavable bond is cleaved, and the capture agent (having its original amino group) is released. The released capture agent has an identical molecular structure to the capture agent reacted with the linking agent. Exemplary photocleavable ortho-nitrobenzyl NHS linkers that may be cleaved by uv light to produce an unaltered cleavage agent are shown in Fig. 3B, 3C, 3D, 3E, 3F and 3G.

Exemplary linking agents that may be employed in the subject methods are described in Guillier et al, *supra* and Olejnik et al (Methods in Enzymology 1998 291:135-154), and further described in U.S.P.N. 6,027,890; Olejnik et al (Proc. Natl. Acad Sci, 92:7590-94 ); Ogata et al. (Anal. Chem. 2002 74:4702-4708); Bai et al (Nucl. Acids Res. 2004 32:535-541); Zhao et al (Anal. Chem. 2002 74:4259-4268); and Sanford et al (Chem Mater. 1998 10:1510-20), and are purchasable from Ambergen (Boston, MA; NHS-PC-LC-Biotin), Link Technologies (Bellshill, Scotland), Fisher Scientific (Pittsburgh, PA; PIERCE EZ-LINK™ NHS-PC-LC-Biotin) and Calbiochem-Novabiochem Corp. (La Jolla, CA).

Many NHS and ortho-nitrobenzyl-group containing linking agents may be used in the subject methods. Representative linking agents have the following structure:

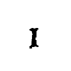

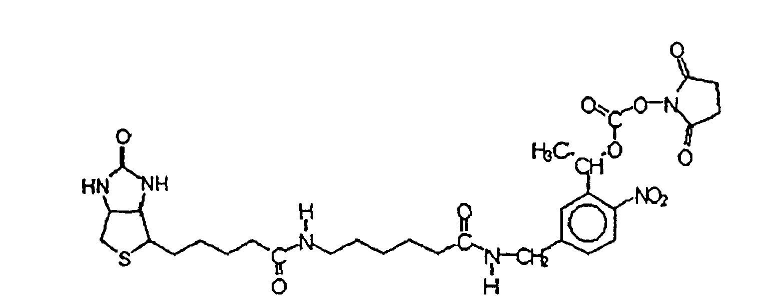

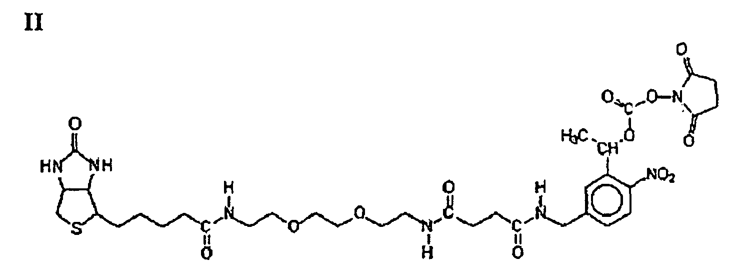

As would be recognized by one of skill in the art, capture agents can be pre-made (e.g., synthesized by a machine or made by recombinant means) and then covalently bound to the linker. Alternatively, capture agents that are already covalently bound to a linker may be made using synthetic means (e.g., using a machine).

In one embodiment, an NHS and ortho-nitrobenzyl group-containing biotinylated linker agent is combined with a primary amine-group containing capture agent (e.g., a polypeptide) at pH 7-9. The amine reacts with the NHS group by nucleophilic attack, and the by-product of the reaction, N-hydroxysuccinimide, is released. The resulting biotinylated photocleavable linker-containing capture agent may be stored in DMF or DMSO protected from light and moisture at -20°C, indefinitely.

The biotinylated photocleavable linker-containing capture agent may be contacted with a transparent substrate coated in streptavidin or avidin and incubated a suitable amount of time (e.g., 15-30 minutes) in binding buffer with gentle mixing. The biotinylated photocleavable linker-containing capture agent thereby becomes bound to the streptavidin. The bound capture agent may be washed in phosphate buffered saline (PBS) or other suitable buffer, and the transparent substrate may then be employed as a component of an evanescent wave sensor.

A non-biotinylated linker agent may be attached to a substrate via reactive functional moieties on both the substrate and the linker. The reactive moiety of the linker agent (e.g., an amino group, a sulfide group, etc.) can be reacted with a suitable site (e.g., a carboxylic acid group, a reactive-halogen, etc.) on the surface of a substrate to produce a covalent bond between the substrate and the non-biotinylated photcleavable linker-containing capture agent. The substrate with the bound capture agent may then be employed as a component of an evanescent wave sensor.

The cleavably bound capture agent may be cleaved from the substrate by exposing the substrate to light of 300-370 nm wavelength, e.g., 365 nm, at a suitable intensity, e.g., 1.2mW/cm².

In an alternative embodiment, a photoacid generator (PAG) material may be employed as a cleavage agent. Such PAGs are known in the art (see, e.g., the world wide website of Sigma-Aldrich) and include N-hydroxyphthalimide trifluoromethanesulfonate, 2-Naphthyl diphenylsulfonium triflate, bis(4-tert-butylphenyl)iodonium perfluoro-1-butanesulfonate, bis(4-tert-butylphenyl)iodoniump-toluenesulfonate, bis(4-tert-butylphenyl)iodonium trifluoromethanesulfonate, (4-Bromophenyl)diphenylsulfonium trifluoromethanesulfonate, (tert-Butoxycarbonylmethoxynaphthyl)-diphenylsulfonium triflate, and many others. In this embodiments, a PAG may present in the flowstream of the sensor (e.g., mixed with sample). When UV light is directed at an area (i.e., a localized region) of this flow stream, protons are generated within the lighted area and the pH of the area drops. Any pH-sensitive material present in the area (e.g., sensitive esters or pH-sensitive binding) would be subject to modification (i.e., cleavage, hydrolysis, binding-disruption). For example, photo-induced acid generation produced by PAG is sufficient to cleave an ester bond to release a capture agent bound to a substrate by such a bond.

In a further embodiment, a capture agent may be bound to a substrate through an electrolytically-cleavable linker. In this case, the capture would be electrochemically releasable from the substrate. In this embodiment, discrete electrodes could be positioned on the substrate at each address of the substrate, and independent application of a electrical potential to those electrodes could cause cleavage of the bound capture agent, or reverse-binding of the capture agent.

Acid-cleavable linkers may also be cleaved by the local, *in situ,* acid generation. Method for performing such method are readily adapted from Donner et al (Biochemica 4, **2003,** a publication of Roche Applied Science, Indianapolis" IN).

### ANALYTE DETECTION SYSTEMS

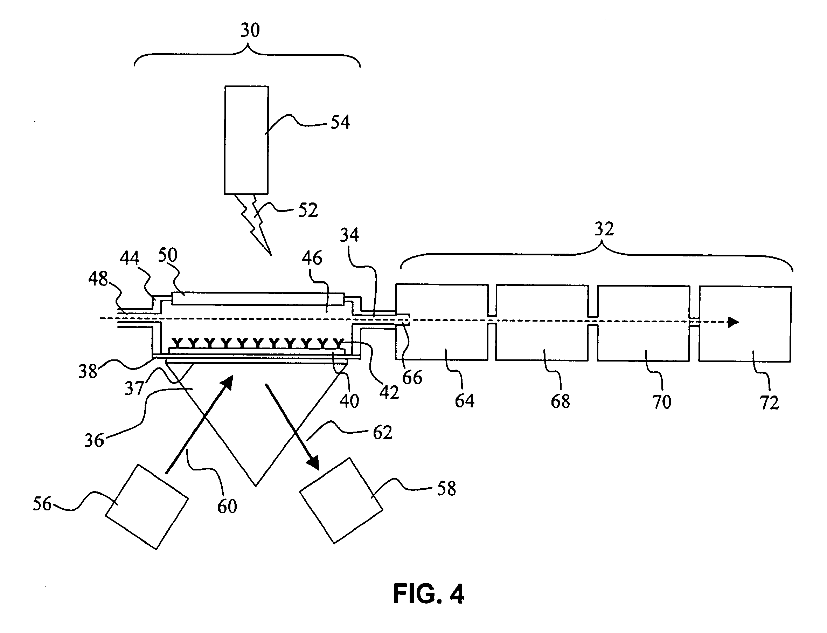

As noted above, the invention provides an integrated analyte detection system. Referring to **Fig. 4**, the system contains two main components, a subject evanescent wave sensor 30 and a mass spectrometry apparatus 32, linked by fluid transport device **34** (e.g. a conduit such as a flexible capillary tube or planar chip). In certain embodiments, the evanescent wave sensor **30,** described in greater detail above, contains prism **36** positioned on optically matched substrates **37** and **38,** metal layer **40,** and cleavably bound capture agents **42.** The capture agents are housed in housing **44** that defines a liquid-tight chamber **46.** Sample is introduced into chamber **46** via sample input device **48,** and exits chamber **46** via fluid transport device **34.** Depending on the choice of cleavage agent (e.g., whether it is a photocleavable agent) the housing may have a light (e.g., uv light) transparent window **50** that permits an external light stimulus **52** to enter chamber **46** and cleave the linkers of any susceptible surface-bound capture agents. Accordingly, in certain embodiments, a subject analyte detection system may contain a first light source **54,** e.g., a laser or the like, for providing a cleaving stimulus to the bound capture agents **42.** A subject system may also contain a second light source **56** and a detector **58** that are under the control of a microprocessor and suitable software. Programming for operating the system may be loaded onto the system, or a computer/microprocessor may be preprogrammed to run with the same.

Light source **56** may be a wavelength-tunable laser. By way of various optics, generally including a collimator, a light beam **60** is directed toward a prism **36.** The beam passes into and through substrate **38** and reflects off metal layer **40.** The resultant signal **62** is collected in photo-detector **58.** In certain embodiments, a collimated beam of light of varying wavelength may be used, and data may be collected using a compound metal oxide semiconductor (CMOS) imager or a charge coupled device (CCD) imager. In this manner, data for an entire sensor or for selected sections of a sensor can be collected simultaneously. If a capture agent is present, beam **60** undergoes total internal reflection within the substrate at its boundary with a metal coating **40,** and a change in signal **62** can be detected by detector **58.**

Mass spectrometry apparatus **32** contains ion source **64** containing ionization device **66** (e.g., an electrospray or MALDI interface), ion transport device **68,** mass spectrometer **70** (e.g., a TOF-MS or quadrupole or triple quadrupole-MS, ion trap-MS) and ion detector **72.**

Fluid transport device **34** may contain appropriate feeds and valves to allow diversion of samples from the mass spectrometer to facilitate washing of the capture agents, removal of undesirable materials from the chamber, and for system flushing etc. Fluid transport device **34** and sample input device **48** may contain various pumps and regulators to control the flow of liquid sample into and out of the components of the subject system.

As would be readily apparent to one of skill in the art, the configuration of the elements shown in Fig. 4 may be altered to provide a system capable of performing the subject methods. For example, the photocleaving light stimulus may be initiated from the prism side of the subject evanescent wave sensor, may pass through and exit the prism, and cleave the linked capture agents after emergence from the prism.

### METHODS OF DETECTING ANALYTES

In use, a subject system may be employed to detect an analyte by its binding to a capture agent and by mass spectrometry. In greater detail, the invention provides a method for assessing the presence of an analyte in a sample, comprising: a) contacting a sample with a capture agent for the analyte that is cleavably bound to a surface of a substrate of an evanescent wave sensor; b) assessing the presence of the analyte on the substrate by detecting an evanescent wave; c) cleaving the capture agent from the surface to produce a released sample comprising the analyte; and d) assessing the presence of the analyte in the released sample by mass spectrometry.

One embodiment of this method may be described with reference to Fig. 4. In Fig. 4, the movement of the analyte (or ion thereof) is indicated by the dotted line arrow.

In general, the subject methods involve contacting a subject sensor with a sample under specific binding conditions and assessing binding of analytes in the sample to the capture agents by evanescent wave detection. In certain embodiments, an evanescent wave is detected by reflecting light off a metal layer, and detecting the angle and/or intensity of the reflected light. In other embodiments, a graphical image of the sensor surface may be produced. Binding of an analyte to capture agents present on the sensor can be detected by evaluating changes in reflected light angle and/or intensity, or changes in the graphical image, for example.

In particular embodiments, a subject sensor may be used in surface plasmon resonance (SPR) methods. SPR may be achieved by using the evanescent wave which is generated when a laser beam, linearly polarized parallel to the plane of incidence, impinges onto a prism coated with a thin metal film. SPR is most easily observed as a change in the total internally reflected light just past the critical angle of the prism. This angle of minimum reflectivity (denoted as the SPR angle) shifts to higher angles as material is adsorbed onto the metal layer. The shift in the angle can be converted to a measure of the thickness of the adsorbed or added material by using complex Fresnel calculations and can be used to detect the presence or absence of analytes bound to the capture agents on top of the metal layer. As is well known, SPR may be performed with or without a surface grating (in addition to the prism). Accordingly a subject sensor may contain a grating, and may be employed in other SPR methods other than that those methods explicitly described in detail herein.

In using SPR to test for binding between agents, a beam of light from a laser source 56 is directed through a prism onto a subject sensor containing a transparent substrate, which has one external surface covered with a thin film of a metal, which in turn is covered with a capture-agent that binds an analyte, as discussed above. The SPR angle changes upon analyte binding to the capture agents. By monitoring either the position of the SPR angle or the reflectivity at a fixed angle near the SPR angle, the presence or absence of an analyte in the sample can be detected.

Various types of equipment for using SPR with a biosensor for biological or biochemical or chemical substances are known in the art (and described by Liedberg et al. (1983) Sensors and Actuators 4:299, European Patent Application 0305108 and U.S. Pat. No. 5,374,563, etc.), including grating coupled systems, optical waveguide systems and prism coupled attenuated total reflection systems.

In certain embodiments, a light source (typically a monochromatic light source) is used to illuminate the prism/metal film at an incident angle that is near the SPR angle, and the reflected light is detected at a fixed angle with a CCD camera to produce an SPR image. The SPR image arises from variations in the reflected light intensity from different parts of the sample; these variations are created by any changes in organic film thickness or changes in index of refraction that occur upon adsorption onto the modified gold surface. SPR imaging is sensitive only to molecules in proximity to the surface, therefore unbound molecules remaining in solution do not interfere with in situ measurements.

In certain embodiments, the angles of incidence and reflection are "swept" together through the resonance angle, and the light intensity is monitored as function of angle. Very close to the resonance angle, the reflected light is strongly absorbed by the gold surface, and the reflected light becomes strongly reduced. In other embodiments, the source and detector angles are fixed near the resonance angle at an initial wavelength, and the wavelength is swept to step the resonance point through the fixed angle. The beam is collimated and an entire image of the substrate is captured. In exemplary embodiments, the wavelength of the tunable laser may be between from 0.6 µm to about 0.8 µm (i.e., having a 200 nm sweep), although tunable lasers having other sweeps (e.g., 0.8 µm to 1.0 µm, 1.0 µm to 1.2 µm, 1.2 µm to 1.4 µm, 1.4 µm to 1.6 µm or 1.6 µm to 1.8 µm may also be employed. In one embodiment, a tunable laser having a sweep of 1.45 to 1.65 µm is employed.

With reference to Fig. 4, a sensor reader is used to accomplish the task of obtaining data from a subject sensor, which readers are generally well known in the art (see U.S. Patent 6,466,323, for example). In one embodiment, capture agents 42 are bound to the substrate. A liquid sample of interest is introduced into chamber 46, and analytes of interest bind to with capture agents for those analytes 42. As a greater number of biomolecules become bound thereto, their mass concentration increases, resulting (for a given incident angle of light in an applied range of beam angles "R") in a light reflectance angle "θ" where light intensity maximizes, minimizes, or varies.

Evanescent wave producing light having a wavelength of between about 400 nm to about 2.0 µm may used in the subject methods. In exemplary embodiments, the wavelength of light used is from about 0.8 µm to about 1.7 µm, e.g., 0.8 µm to about 1.6 µm. In certain embodiments, the light used is monochromatic light, and the light may be polarized, and in certain embodiments, the wavelength of light used may change, i.e., may "sweep" during reading of a sensor. Accordingly, the light used may not be of a static wavelength. In typical embodiments, the wavelength may sweep between two different wavelengths separated by about 100 nm, about 200 nm, about 300 nm or about 400 nm or more, with the lower wavelength being any of the wavelengths listed above. The light employed in for evanescent wave detection should have a wavelength that is different to that of the light used as a cleaving stimulus, and, as such, should not cleave the capture agents from the substrate. In one embodiment, the evanescent wave-producing light has a wavelength of 1.45 µm to 1.65 µm may be employed, although broadband evanescent wave-producing light of a wide variety of sweeps and wavelengths may be used.

If a light-cleavable linker is employed in the subject method, the light employed for SPR analysis should be at a wavelength that does not cleave the linker. In other words, the light employed for SPR analysis should be compatible with the cleavable linker.

Once binding of an analyte to a capture agent has been detected using such an evanescent wave detector, the capture agent and the analyte bound thereto may be separated from the substrate by cleaving the cleavable linker attaching the capture agent to the substrate, as discussed above. The separated capture agent and bound analyte may then be transported through fluid transport device **34** (typically using a pump) and subjected to mass analysis in mass spectrometry system **32.**

In general, mass analysis involves transporting a sample, generally but not always present in a solvent, into ion source **64** of a mass spectrometer system where the constituents of the sample are ionized. Sample ionization may be performed using a variety of means, including electrospray ionization (ESI), an atmospheric pressure chemical ionization (APCI), atmospheric pressure photoionization (APPI) and matrix-assisted ionization (e.g., MALDI). As would be recognized by one of skill in the mass spectrometry arts, the transfer of a sample from the chamber of a subject evanescent wave sensor into an ion source and its subsequent ionization is akin to the transfer of a sample from a liquid chromatography system into an ion source of a mass spectrometer, and ionization of the sample therein.

Upon ionization of the analytes of a sample, the analyte ions are generally transferred through at least one vacuum chamber **68** to a mass spectrometer **70** and then to detector **72.** These devices are well known components of conventional mass spectrometry systems and need not be discussed herein in any great detail.

A variety of different mass analyzers may be a part of the above described system, including time of flight (TOF), Fourier transform ion cyclotron resonance (FTICR), ion trap, quadrupole or double focusing magnetic electric sector mass analyzers, or any hybrid thereof.

In certain embodiments, ion source **64** is at ambient pressure, intermediate vacuum chamber **68** is held at a pressure that is around two orders of magnitude less than the ambient pressure, and mass analyzer **70** is held at a pressure of around two to four orders of magnitude less than that of the intermediate chamber. Ions leaving the ionization device **66** are directed towards and enter a sampling device and are swept into the first vacuum chamber **68** in a stream of gas due to the pressure difference between ion source **64** and chamber **68.** The ions exit chamber **68** and enter mass analyzer **70.** Mass analyzer **70** separates on the basis of their m/z ratio of the ions, and those ions are detected by detector **72.**

### UTILITY

The subject methods and compositions find use in a variety applications, where such applications are generally analyte detection applications in which the presence of a particular analyte in a given sample is detected at least qualitatively, if not quantitatively. Protocols for carrying out SPR assays are well known to those of skill in the art and need not be described in great detail here. Generally, the sample suspected of comprising the analyte of interest is contacted a subject sensor under conditions sufficient for the analyte to bind to its respective binding pair member that is present on the sensor. Thus, if the analyte of interest is present in the sample, it binds to the sensor at the site of its complementary binding member and a complex is formed on the sensor surface. The presence of this binding complex on the surface of the sensor is then detected using SPR.

Specific analyte detection applications of interest include hybridization assays in which the nucleic acid capture agents are employed and protein binding assays in which polypeptides, e.g., antibodies or peptides, are employed. In these assays, a sample is first prepared and following sample preparation, the sample is contacted with a subject sensor under specific binding conditions, whereby complexes are formed between target nucleic acids or polypeptides (or other molecules) that are complementary to probe sequences attached to the sensor surface. The presence of complexes is then detected using SPR.

Results from reading a subject sensor and/or mass spectrometer apparatus may be raw results or may be processed results such as obtained by applying saturation factors to the readings, rejecting a reading which is above or below a predetermined threshold and/or any conclusions from the results (such as whether or not a particular analytes may have been present in the sample). The results of the reading (processed or not) may be forwarded (such as by communication) to a remote location if desired, and received there for further use (such as further processing). Stated otherwise, in certain variations, the subject methods may include a step of transmitting data from at least one of the detecting and deriving steps, to a remote location. The data may be transmitted to the remote location for further evaluation and/or use. Any convenient telecommunications means may be employed for transmitting the data, e.g., facsimile, modem, Internet, *etc.* Alternatively, or in addition, the data representing results may be stored on a computer-readable medium of any variety such as noted above or otherwise. Retaining such information may be useful for any of a variety of reasons as will be appreciated by those with skill in the art.

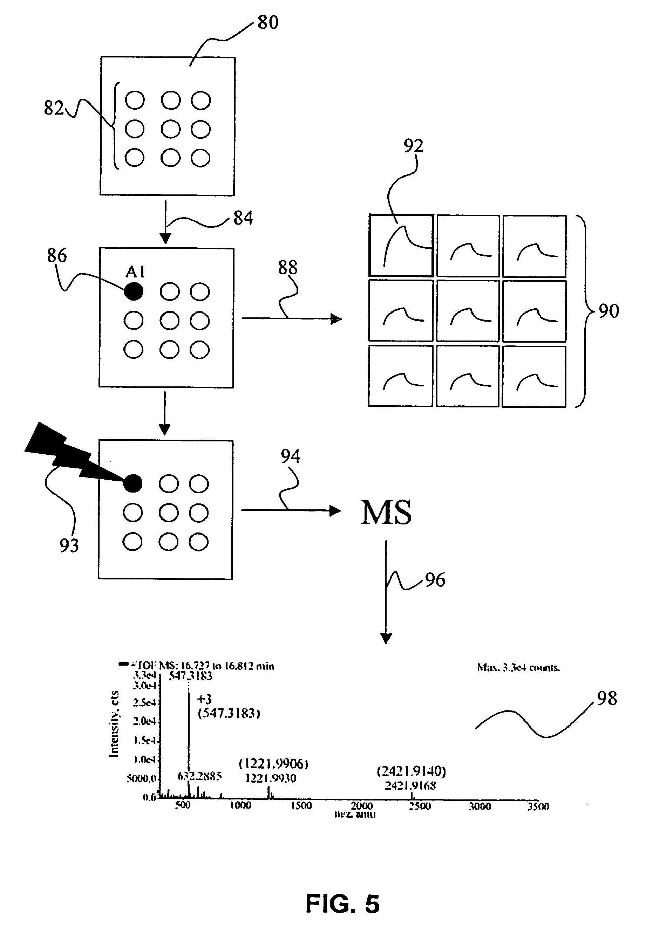

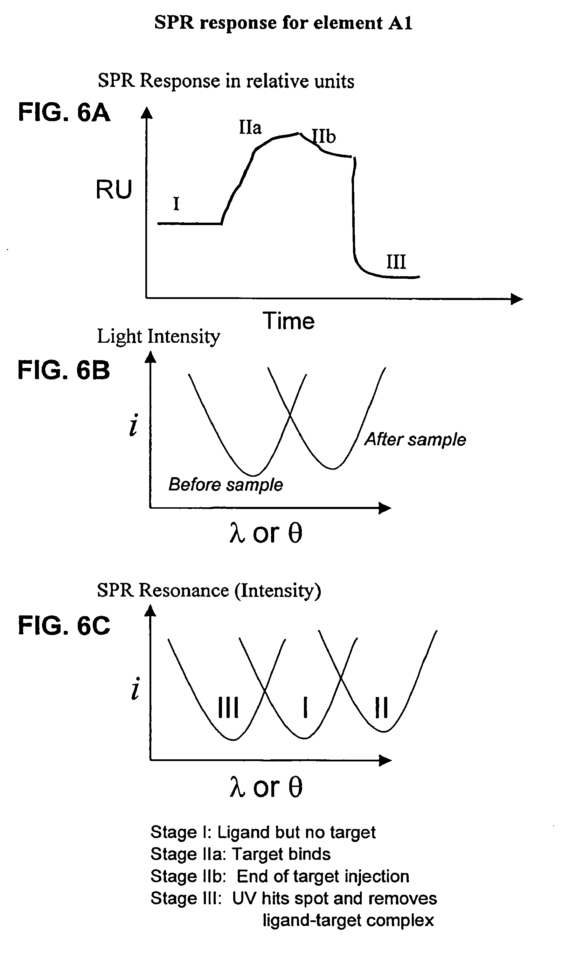

One embodiment of particular interest is illustrated in Fig. 5. In this embodiment, subject evanescent wave sensor **80** contains an addressable array of different capture agents **82** bound to its surface. The array of capture agents **82** of evanescent wave sensor **80** are contacted with a sample under conditions suitable for binding of the analytes in the sample to the capture agents (which conditions may include washing). In this illustrated example, the capture agent feature at position "A1" of the array binds an analyte, and the array is read by evanescent wave detection **88.** Illustrative results of reading of the array by evanescent wave detection **90** indicate binding of an analyte to the capture agent present in feature A1 because the reflected light signal corresponding to that feature **92** is different to the signals for other features. Upon detection of analyte binding to a particular feature, that feature but not other features is particularly subjected to a stimulus **93** (using, for example, a laser or the like) that releases the capture agents in that feature (but not the capture agents of other features), and the analytes bound thereto. The released capture agents and analytes are transferred into a mass spectrometry system **94** and subject to mass analysis 96. Exemplary results obtained from mass analysis **98** may be used investigate the bound analyte. For example, the results may be used to confirm the identify of the capture agent-bound analyte, or elucidate the molecular structure of the capture agent-bound analyte is the structure is not known. Exemplary SPR response data for element A1 are shown in Figs. 6A-6C.

Accordingly, the inventions provides "addressable recovery" of capture agent-bound analytes from a subject sensor, where "addressable recovery" is the exclusive release of capture agent-bound analytes a particular, defined, area of a subject sensor, e.g., a feature of a capture agent array.

### KITS

Also provided by the subject invention are kits for practicing the subject methods, as described above. The subject kits at least include a substrate and a capture agent cleavable linked to a surface of the substrate. Alternatively, the kit may include a substrate and a cleavable linker on its surface with directions for the user to bind a capture agent of choice. The various components of the kit may be present in separate containers or certain compatible components may be precombined into a single container, as desired.

In addition to above-mentioned components, the subject kits typically further include instructions for using the components of the kit to practice the subject methods. The instructions for practicing the subject methods are generally recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or subpackaging) etc. In other embodiments, the instructions are present as an electronic storage data file present on a suitable computer readable storage medium, e.g. CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, this means for obtaining the instructions is recorded on a suitable substrate.

In addition to the subject database, programming and instructions, the kits may also include one or more control analyte mixtures, e.g., two or more control analytes for use in testing the kit.

### EXAMPLES

The following examples are put forth so as to provide those of ordinary skill in the art with a complete disclosure and description of how to make and use the present invention, and are not intended to limit the scope of what the inventors regard as their invention. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1

### Synthesis of a photoactive linker: (4-aminomethyl-3-nitrobenzoic acid, methyl ester-HCl salt)

To 4-(Bromomethyl)-3-nitrobenzoic acid (Aldrich # 423564 -1 g, 3.8 mmol) in 10 ml of 2,2-dimethoxypropane (Aldrich # D13680-8) was added 0.95 ml of concentrated hydrochloric acid. The solution was stirred overnight at room temperature for 20 hr. The solvents were stripped in vacuo and the residue placed into equal volumes of ethyl acetate and water. After separation, the organic layer was washed with 2N HCl, saturated NaHCO₃ solution, then brine and dried over Na₂SO₄. Removal of solvents gave a solid that was triturated with hexane, then ether. ES-MS shows methyl ester product: m/z = 274 [M + H]+ containing 1 Bromine.

This ester (0.38g, 1.4 mmol) was added to a solution of 0.21 (1.5 mmol) of hexamethylenetetramine (Aldrich # 398160) in 15-20 ml of chlorobenzene. The milky-yellow solution was stirred overnight at room temperature. Then the solution and its precipitate were centrifuged (15 min at 2000 rpm). After decanting, the material was again centrifuged with chlorobenzene, then ether (2x). The ether washes were colorless. The precipitate was dried at room temperature to a pale yellow powder (0.46g, quant.). ES-MS m/z = 211 [M - amine salt] ⁺.

The intermediate ester-salt (0.46g, 1.4 mmol) was placed into 28.5 ml of ethanol and 1.5 ml water and then concentrated HCl (0.62 ml, 7.5 mmol, 5 eq.) was added. This mixture was stirred overnight at room temperature. Removal of solvents left a yellowish solid that was the end product-photoactive linker (0.4 g).

### EXAMPLE 2

### Binding of Photoactive Linker to Carboxy-Terminated Substrates.

A carboxy-terminated substrate was prepared by applying a thin film of gold to a piece of glass. A self-assembled monolayer was attached to the gold film using 11-mercapto-undecanoic acid (Aldrich # 450561). This carboxy-terminated substrate was covered with a solution of 2 mg of the photoactive linker, 19.2 mg of EDC (Aldrich # 161462, 1-[3-(Dimethylamino)propyl]-3-ethylcarbodiimide hydrochloride) dissolved in 1 ml of 100 mM phosphate buffer, pH 5. The substrate and solution was left in the dark overnight at room temperature, then rinsed with deionized water and dried with a stream of nitrogen. Contact angle = 52-57 degrees.

The substrate-linker was then hydrolyzed in the dark to a terminal carboxylic acid with overnight treatment of a methanol-water solution brought to a pH of 12.8 with sodium hydroxide. Again, substrate was rinsed thoroughly with DI water and dried with a stream of nitrogen. Contact angle =10.5 degrees.

### EXAMPLE 3

### Binding and Photolysis of Strepavidin-cy3 (Ligand) to Carboxy-Terminated Substrate-Linker.

The substrate-linker was spotted (10-15 ul/spot) with a solution of 9.6 mg of EDC and 7 u1 of strepavidin-cy3 conjugate (Sigma # S-6402) dissolved in 0.5 ml of 100 mM phosphate buffer, pH 5. The substrate was left in the dark overnight at room temperature, then rinsed with deionized water and dried with a stream of nitrogen. Fluorescent counts on spots: 1200-1700. Background fluorescence: 50 counts.

Spotted substrate was photolyzed at 254nm for 90 min in water or buffers to remove fluorescent ligand. Fluorescent counts: 50, Background counts: 50.

The above discussion demonstrates an analyte detection system containing a cleavably-bound capture agent evanescent wave sensor and a mass spectrometer. The system facilitates the detection of analytes by detecting a evanescent wave, and investigating the identity of those analytes by mass spectrometry. Accordingly, the subject system represents a significant contribution to the art.

All publications and patents cited in this specification are herein incorporated by reference as if each individual publication or patent were specifically and individually indicated to be incorporated by reference. The citation of any publication is for its disclosure prior to the filing date and should not be construed as an admission that the present invention is not entitled to antedate such publication by virtue of prior invention.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. An evanescent wave sensor comprising:
a substrate; and
a capture agent,
wherein said capture agent is cleavably bound to a surface of said substrate in said evanescent wave sensor.

2. The evanescent wave sensor of claim 1, wherein said linker is a photocleavable linker

3. The evanescent wave sensor of claim 1, wherein said linker is a uv light-cleavable linker.

4. The evanescent wave sensor of claim 1, wherein said capture agent can be cleaved from said surface without altering the native structure of said capture agent.

5. The evanescent wave sensor of claim 1, wherein said cleavable linker comprises a photocleavable ortho-nitrobcnzylic group.

6. The evanescent wave sensor of claim 1, wherein said sensor is operably connected to an ion source of a mass spectrometry apparatus.

7. The evanescent wave sensor of claim 1, wherein said sensor is comprised with a housing that is at least partly uv-transparent.

8. A system for detecting analytes, comprising:
an evanescent wave sensor comprising:
a) a substrate; and
b) a capture agent that is cleavably bound to a surface of said substrate;
an ion source of a mass spectrometry apparatus operably connected to said evanescent wave sensor;
a mass spectrometer; and
a mass detector.

9. A method for assessing the presence of an analyte in a sample, comprising:
contacting said sample with a capture agent for said analyte that is cleavably bound to a surface of a substrate of an evanescent wave sensor;
assessing the presence of said analyte on said substrate by detecting an evanescent wave;
cleaving said capture agent from said surface to produce a released sample comprising said analyte; and
assessing the presence of said analyte in said released sample by mass spectrometry.
